# FASCICULE DE BREVET EUROPEEN

(11) **EP 1 968 925 B1**
(45) Date de publication et mention de la délivrance du brevet: **04.11.2015**
(21) Numéro de dépôt: 06847065.7
(22) Date de dépôt: 19.12.2006
(51) Int. Cl.: C07C 29/149, C07C 31/38

(54) **PROCEDE DE PREPARATION DE DIFLUOROETHANOL**
HERSTELLUNGSVERFAHREN FÜR DIFLUORETHANOL
PROCESS FOR PRODUCING DIFLUOROETHANOL

(30) Priorité: 19.12.2005 FR 0512902
(43) Date de publication de la demande: 17.09.2008
(73) Titulaire: Rhodia Opérations, 75009 Paris (FR)
(72) Inventeur: BUISINE, Olivier, F-69230 Saint Genis Laval (FR); JACQUOT, Roland, F-69340 Francheville (FR)
(74) Mandataire: Menville, Laure
(86) Numéro de dépôt international: PCT/FR2006/002780
(87) Numéro de publication internationale: WO 2007/071841

(56) Documents cités:
- EP-A1- 1 224 971
- PATENT ABSTRACTS OF JAPAN vol. 011, no. 133 (C-418), 25 avril 1987 (1987-04-25) & JP 61 268639 A (ASAHI GLASS CO LTD), 28 novembre 1986 (1986-11-28) cité dans la demande
- A.L. HENNE ETAL.: "Acidity of trifluorinated alcohols and saponification rates of their acetates." JOURNAL OF THE AMERICAN CHEMICAL SOCIETY, vol. 74, 1952, pages 1426-1428, XP002393955 cité dans la demande

## Description

La présente invention a pour objet un nouveau procédé de préparation de difluoroéthanol.

Le difluoroéthanol est un alcool utilisé dans la synthèse organique, notamment dans le domaine de l'agrochimie et de la pharmacie.

On a déjà proposé différents procédés de synthèse du difluoroéthanol.

On peut notamment produire du difluoroéthanol par réduction du chlorure de difluoroacétyle, soit par des hydrures [AL. Henne, RL. Pelley - JACS 74 (1952), 1426-8], soit par hydrogénation catalytique [Asahi Chemical - brevet JP61268639 du 24/05/85], par réduction du difluoroacétate d'éthyle à l'aide de borohydrure de sodium [M. Lewis, E. de Clerck - J. Chem. Res. Miniprint 8 (2001), 844-56].

On peut également citer la réduction de l'acide difluoroacétique par le complexe borane/diméthylsulfure [W.G. Reifenrath, E.B. Roche, J. Med. Chem. 23(9), (1980), 985-90].

Toutefois, ces procédés nécessitent plusieurs étapes de synthèse à partir de l'acide difluoroacétique ou mettent en oeuvre des réactifs onéreux qui en font des procédés complexes et pas économiquement viables

Une autre voie d'accès au difluoroéthanol consiste à effectuer l'hydrolyse de 2,2-difluoro-1-bromoéthane entre 150 et 200°C. Les rendements annoncés sont de 50 % environ.

L'objectif de la présente invention est de fournir un procédé de préparation de difluoroéthanol à partir d'un réactif aisément accessible.

Il a maintenant été trouvé et c'est ce qui constitue l'objet de la présente invention, un procédé de préparation de difluoroéthanol caractérisé par le fait qu'il comprend une hydrogénation, en présence d'une quantité efficace d'un catalyseur comprenant au moins un élément du groupe VIII de la classification périodique déposé sur un support minéral solide à propriétés acides, d'un halogénure d'acétyle répondant à la formule suivante : 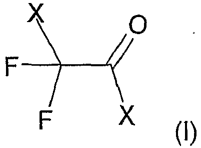 dans ladite formule, X représente un atome d'halogène choisi parmi un atome de chlore et un atome de brome.

Le procédé de l'invention s'applique tout particulièrement à un substrat répondant à la formule (I) dans laquelle X représente un atome de chlore.

Conformément au procédé de l'invention, on effectue une triple hydrogénation catalytique à savoir la réduction du groupe carbonyle et une double deshydrohalogénation.

Comme catalyseurs d'hydrogénation préférés, on met en oeuvre un élément métallique choisi parmi les éléments du groupe VIII de la Classification périodique des éléments.

Pour la définition des éléments, on se réfère ci-après à la Classification périodique des éléments publiée dans le Bulletin de la Société Chimique de France, n°1 (1966).

Il s'agit plus particulièrement des métaux nobles tels que : le ruthénium, le rhodium, le palladium, l'osmium, l'iridium, le platine.

On peut également utiliser un mélange desdits éléments.

Comme éléments métalliques mis en oeuvre préférentiellement, on peut citer le palladium et/ou le platine.

Le métal peut être déposé sur le support sous forme d'un métal finement divisé ou sous la forme d'un composé qui sera réduit en métal en présence d'hydrogène. Ainsi, on peut le mettre en oeuvre sous forme d'un dérivé inorganique tel qu'un oxyde ou un hydroxyde. Il est possible de faire appel à un sel minéral de préférence, nitrate, sulfate, oxysulfate, halogénure, oxyhalogénure, silicate, carbonate, ou à un dérivé organique de préférence, cyanure, oxalate, acétylacétonate ; alcoolate et encore plus préférentiellement méthylate ou éthylate ; carboxylate et encore plus préférentiellement acétate. Peuvent être également mis en oeuvre des complexes, notamment chlorés dudit métal noble et/ou de métaux alcalins, de préférence sodium, potassium ou d'ammonium

Le choix du support est conditionné par le fait qu'il doit s'agir d'un solide minéral à propriétés acides de Lewis ou pouvant développé une acidité de Lewis en présence d'acide chlorhydrique libéré dans le procédé de l'invention.

Pour la définition d'un acide de Lewis, on peut se référer, entre autres, à l'ouvrage de Jerry MARCH, ADVANCED ORGANIC CHEMISTRY, 8, p.260 (1992).

L'acide de Lewis est défini comme toute espèce chimique présentant une orbitale vacante et, par conséquent susceptible d'accepter des électrons.

Le support du catalyseur pour l'invention est choisi parmi l'alumine et les silico-aluminates naturels ou synthétiques.

L'alumine est le support choisi préférentiellement dans le procédé de l'invention. Il peut s'agir de l'alumine α, γ, η. L'alumine γ est préférée.

En ce qui concerne les silico-aluminates naturels ou synthétiques on peut mentionner les argiles acides et les zéolithes acides.

Un premier type de supports minéraux convenant tout à fait bien à la mise en oeuvre du procédé de l'invention sont les argiles acides.

Comme exemples préférés, on peut citer les montmorillonites. Elles peuvent être représentées par la formule 3SiO₂, Al₂O₃, (Mg,Ca)O, nH₂O avec n compris entre 5 et 7. On peut également mentionner la bentonite.

On fait appel préférentiellement aux argiles commerciales déjà acides telles que notamment, les argiles suivantes : TONSIL OPTIMUM FF et K 10.

On peut traiter si nécessaire, les argiles commerciales, à l'aide d'une solution aqueuse d'un acide fort. Le traitement est effectué par l'Homme du Métier, d'une manière classique. On peut par exemple introduire l'argile dans une solution aqueuse d'acide sulfurique à pH compris entre 2 et 4.

Comme autres supports convenant à l'invention, on peut mentionner les zéolithes.

Les zéolithes naturelles sont des matériaux appartenant à la famille des alumino-silicates hydratés de métaux des groupes IA et IIA de la classification périodique des éléments (par exemple : calcium, magnésium, potassium).

Structurellement, les zéolithes sont complexes, polymères inorganiques cristallins basés sur une suite infinie tridimensionnelle de structures quadri-connectées de AlO₄ et de SiO₄ tétraédriques liées entre elles par un échange d'ions oxygène. Chaque AlO₄ tétraédrique présent dans la structure apporte une forte charge négative qui est contre-balancée par un cation (Ca²⁺, Mg²⁺, ...).

Il existe deux sortes de zéolithes : les zéolithes naturelles et les zéolithes synthétiques qui toutes deux, peuvent intervenir dans le procédé de l'invention.

Comme exemples de supports zéolithiques convenant à la présente invention, on peut citer les zéolithes naturelles telles que par exemple : la chabazite, la clinoptilolite, l'érionite, la mordénite, la phillipsite, l'offrétite.

Conviennent préférentiellement à la mise en oeuvre de l'invention, les zéolithes synthétiques. On choisit tout particulièrement les zéolithes telles que la zéolithe HZSM-5 de type MFI, la zéolithe HZSM-11 de type MEL, la zéolithe HY de type faujasite (FAU), la H-mordénite (MOR), la zéolithe KL.

Les zéolithes sont des produits connus et largement décrits dans la littérature, en particulier dans " Atlas of zeolites structure types by W. M. Meier and D. H. Olson published by the Structure Commission of the International Zeolite Association (1992), p. 132. "

On préfère mettre en oeuvre dans le procédé de l'invention les zéolithes synthétiques et plus particulièrement les zéolithes commerciales qui sont sous les formes suivantes :
- les zéolithes HZSM-5 ou silicalite d'aluminium de rapport molaire Si/Al de 10 à 500;
- la zéolithe ZSM-11 de rapport atomique Si/Al de 5 à 30,
- les zéolithes HY de rapport atomique Si/Al de 2,3 à 40,
- la H-mordénite de rapport atomique Si/Al de 5 à 45.

On fait appel préférentiellement aux zéolithes H-Y ou H-Mordénite du commerce sous forme acide telles que les zéolithes de type H-Y telles que CBV 720, de rapport atomique Si/Al respectivement égal à 15, commercialisées par la Société Zeolyst et aux zéolithes de type H-Mordénite telles que CBV 20 A et 90 A de rapport atomique Si/Al égal respectivement à 10 et 45.

Conformément à l'invention, le catalyseur comprend un élément métallique du groupe VIII déposé sur les supports tels que précédemment définis.

Le dépôt sur le support est effectué selon les techniques couramment utilisées par l'Homme du Métier, en particulier l'imprégnation sur support.

Généralement, le métal est déposé à raison de 0,1 % à 10 %, de préférence de 0,5 % à 5 % du poids du catalyseur.

On utilise de préférence, des catalyseurs à base de platine et/ou de palladium, déposés sur support alumine.

De préférence, le platine et/ou le palladium est déposé sur ledit support à raison de 0,5 % à 5 % du poids du catalyseur.

Le catalyseur peut être mis en oeuvre sous forme d'une poudre, de pellets ou bien de granulés.

Conformément au procédé de l'invention, on effectue une hydrogénation catalytique du substrat de départ.

La quantité d'hydrogène mise en oeuvre est d'au moins 2 moles par mole de substrat. Elle se situe de préférence entre 2 et 10 et préférentiellement entre 3 et 10.

Le procédé de l'invention peut être mis en oeuvre en phase vapeur ou en phase liquide.

Un mode de réalisation préféré de l'invention consiste à conduire le procédé de l'invention en phase vapeur.

La température est alors choisie dans une gamme de températures allant de 150°C à 350°C et plus particulièrement entre 200°C et 300°C.

La réaction se déroule sous pression d'hydrogène allant d'une pression légèrement supérieure à la pression atmosphérique jusqu'à une pression de quelques bars. Avantageusement, la pression d'hydrogène varie entre 1 et 10 bars et plus préférentiellement entre 1 et 5 bars.

Un mode de réalisation préféré de l'invention consiste à mettre en oeuvre le procédé selon la technique de lit fixe.

D'une manière préférée, on conduit la réaction en continu, dans un réacteur tubulaire équipé de moyens de chauffage, comportant le catalyseur solide disposé en lit fixe.

Le lit catalytique est porté à la température de réaction choisie dans la gamme définie ci-dessus sur lequel on fait passer le courant d'hydrogène.

Le composé de formule (I) est ensuite envoyé sur le lit catalytique.

Il peut également être introduit dans un solvant tel que défini ci-après.

En phase gazeuse, le temps de séjour du flux de matière sur le lit catalytique est très court et varie généralement entre moins d'une seconde à 1 min environ : la pression étant aussi proche de la pression atmosphérique mais il est possible de travailler sous pression pouvant atteindre 10 bars.

Après passage sur le lit fixe, le flux gazeux est condensé par exemple à une température comprise entre 20°C et 40°C.

On obtient une phase liquide comprenant le difluoroéthanol qui peut être récupéré de manière classique, par distillation ou bien par extraction liquide-liquide à l'aide d'un solvant non miscible, par exemple le diisopropyléther.

Le flux non condensé est envoyé sur une colonne d'abattage à la soude pour éliminer l'acide halohydrique formé au cours de la réaction. Ainsi, l'hydrogène épuré peut être recyclé à la réaction.

Selon un autre mode de réalisation de l'invention, il est possible de conduire la réaction en phase liquide.

La température de la réaction est alors choisie avantageusement entre 20°C à 150°C et de préférence entre 40°C et 70°C.

La réaction se déroule sous pression d'hydrogène allant d'une pression légèrement supérieure à la pression atmosphérique jusqu'à une pression de plusieurs dizaines de bars. Avantageusement, la pression d'hydrogène varie entre 1 et 50 bars et plus préférentiellement entre 10 et 20 bars.

La réaction peut avoir lieu en l'absence de solvant. Toutefois, il est préférable d'opérer en présence d'un solvant. On peut notamment utiliser de l'eau ou un composé organique inerte, tel qu'un hydrocarbure aliphatique ou cycloaliphatique halogéné ou non, de préférence l'hexane, le cyclohexane, le méthylcyclohexane ou un hydrocarbure aromatique halogéné ou non de préférence le toluène, le monochlorobenzène.

Selon un mode discontinu, on charge dans le réacteur, le solvant éventuel, le composé de formule (I) et le catalyseur.

La quantité de catalyseur d'hydrogénation mis en oeuvre, exprimée en poids de catalyseur par poids de composé de formule (I) peut varier, par exemple, entre 0,5 et 20 %, de préférence entre 0,5 et 5 %.

On chauffe à la température définie ci-dessus.

On se met sous la pression d'hydrogène souhaitée et on la maintient en ajoutant de l'hydrogène en continu.

On maintient le milieu réactionnel sous agitation jusqu'à cessation de la consommation d'hydrogène.

Il peut être judicieux tout en maintenant la pression constante dans le réacteur, d'effectuer une purge en continu ou des purges en discontinu pour éliminer l'acide halohydrique formé au cours de la réaction. Ainsi, celui peut être récupéré dans une colonne d'abattage à la soude.

En fin de réaction, on sépare le catalyseur selon les techniques classiques de séparation solide/liquide, de préférence par filtration.

On récupère le produit obtenu d'une manière classique, de préférence par distillation ou par extraction liquide-liquide.

On donne ci-après, un exemple de réalisation de l'invention donné à titre illustratif.

Dans les exemples, le taux de conversion correspond au rapport entre le nombre de substrat transformées et le nombre de moles de substrat engagées et le rendement donné correspond au rapport entre le nombre de moles de produit formées et le nombre de moles de substrat engagées.

### Exemple 1

Dans un réacteur tubulaire en nickel de diamètre interne de 2,54 cm et équipé d'une grille permettant de retenir le catalyseur, on introduit 20 ml de catalyseur composé de 2,5 % en poids de palladium supporté sur des billes d'alumine de type alpha.

L'alumine est de type alpha et se trouve sous forme de billes de diamètre 3 mm.

Au dessus du catalyseur, on introduit 10 ml de poudre de verre pour vaporiser et mélanger les réactifs avant passage sur le catalyseur.

Le réacteur est alors chauffé à 300°C à l'aide d'un four électrique sous un courant de 5 l/h d'hydrogène.

On maintient pendant 30 min dans ces conditions et l'on injecte alors avec une pompe le chlorure de chlorodifluoroacétyle à un débit de 10g/h, tout en maintenant le débit d'hydrogène.

L'hydrogénat est alors condensé dans un récepteur plongé dans un bain d'eau.

Une partie de l'acide chlorhydrique formé est entraîné par le courant d'hydrogène en excès.

Apres 10 heures de fonctionnement dans ces conditions, on trouve après dosage de l'hydrogénât par chromatographie en phase gazeuse que la conversion du chlorure de chlorodifluoroacétyle est de 85 % et que le rendement en difluoroéthanol est de 75 %.

### Exemple 2

Dans un réacteur en Hastelloy B2 de 300 ml, on introduit 100 g de chlorure de chlorodifluoroacétyle, 50 g de cyclohexane et 4 g de catalyseur à 3 % en poids de palladium déposé sur noir de carbone sec.

On purge le réacteur 2 fois avec de l'azote sous 10 bars.

Puis, avec 2 fois de l'hydrogène sous 10 bars, on pressurise ensuite le réacteur sous 15 bars, on agite et l'on chauffe à 75°C.

On maintient la pression constante dans le réacteur pendant toute la durée de la réaction.

Après 5 heures, la consommation d'hydrogène cesse.

On purge le réacteur, on filtre le catalyseur et l'on analyse le milieu réactionnel.

La conversion est totale et le rendement en difluoroéthanol est de 60 %.

Dans ces conditions, on forme un mélange d'esters composé de difluoroacétate de difluoroéthyle et de chlorodifluoroacétate de difluoroéthyle.

### Exemple 3

On reproduit l'exemple 1 mais en utilisant comme catalyseur, du palladium déposé sur zéolithe HY à raison de 3 % en poids. Dans ce cas, la conversion est de 95 % et le rendement en difluoroéthanol est de 78 %.

### Exemple 4

On reproduit l'exemple 1 mais en utilisant comme catalyseur, du palladium déposé sur zéolithe HZSM5 à raison de 3 % en poids. Dans ce cas, le taux de conversion est de 98 % et le rendement en difluoroéthanol est de 70 %.

### Exemple 5

On reproduit l'exemple 1 à l'identique mais en travaillant à 350°C.

On obtient pour un taux de conversion de 100 %, un rendement de 61 %.

## Revendications

1. Procédé de préparation de difluoroéthanol **caractérisé par le fait qu'**il comprend une hydrogénation, en présence d'une quantité efficace d'un catalyseur comprenant au moins un élément du groupe VIII de la classification périodique déposé sur un support minéral solide à propriétés acides choisi parmi l'alumine et les silico-aluminates naturels ou synthétiques, d'un halogénure d'acétyle répondant à la formule suivante : 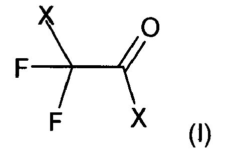 dans ladite formule, X représente un atome d'halogène choisi parmi un atome de chlore et un atome de brome.

2. Procédé selon la revendication 1 **caractérisé par le fait que** l'halogénure d'acétyle mis en oeuvre répond à la formule (I) dans laquelle X représente un atome de chlore.

3. Procédé selon l'une des revendications 1 à 2 **caractérisé par le fait que** le catalyseur est à base d'un élément métallique choisi parmi : le ruthénium, le rhodium, le palladium, l'osmium, l'iridium, le platine.

4. Procédé selon l'une des revendications 1 à 3 **caractérisé par le fait que** l'élément métallique peut être déposé sur le support sous forme d'un métal finement divisé ou sous la forme d'un composé qui sera réduit en métal en présence d'hydrogène.

5. Procédé selon l'une des revendications 1 à 4 **caractérisé par le fait que** le support est l'alumine α, γ, η.

6. Procédé selon l'une des revendications 1 à 4 **caractérisé par le fait que** le support est une argile acide.

7. Procédé selon l'une des revendications 1 à 4 **caractérisé par le fait que** le support est une zéolithe naturelle ou synthétique acide.

8. Procédé selon la revendication 1 **caractérisé par le fait que** le catalyseur est un catalyseur à base de palladium et/ou le platine déposé sur alumine.

9. Procédé selon l'une des revendications 1 à 8 **caractérisé par le fait que** la réaction est conduite en phase vapeur.

10. Procédé selon la revendication 9 **caractérisé par le fait que** la réaction est conduite à une température choisie dans une gamme de températures allant de 150°C à 350°C.

11. Procédé selon l'une des revendications 9 à 10 **caractérisé par le fait que** la réaction se déroule sous pression d'hydrogène variant entre 1 et 10 bars.

12. Procédé selon l'une des revendications 9 à 11 **caractérisé par le fait que** le temps de séjour du flux de matière sur le lit catalytique est très court et varie entre moins d'une seconde à 1 min.

13. Procédé selon l'une des revendications 9 à 12 **caractérisé par le fait que** la réaction est conduite selon la technique du lit fixe.

14. Procédé selon la revendication 13 **caractérisé par le fait que** l'on porte le lit catalytique à la température de la réaction, l'on fait passer le courant d'hydrogène puis l'on introduit le composé de formule (I).

15. Procédé selon l'une des revendications 1 à 7 **caractérisé par le fait que** la réaction est conduite en phase liquide.

16. Procédé selon la revendication 15 **caractérisé par le fait que** la pression d'hydrogène est comprise entre 1 et 50 bars.

17. Procédé selon l'une des revendications 15 à 16 **caractérisé par le fait que** la réaction est conduite à une température choisie dans une gamme de température allant de 20°C à 150°C.

18. Procédé selon l'une des revendications 15 à 17 **caractérisé par le fait que** la réaction est conduite dans un solvant organique choisi parmi les hydrocarbures aliphatiques, cycloaliphatiques, aromatiques halogénés ou non.

19. Procédé selon l'une des revendications 15 à 18 **caractérisé par le fait que** la quantité de catalyseur d'hydrogénation mis en oeuvre, exprimée en poids de catalyseur par poids de composé de formule (I) varie entre 0,5 et 20 %.

20. Procédé selon l'une des revendications 1 à 19 **caractérisé par le fait que** l'on récupère le difluoroéthanol en fin de réaction par distillation ou par extraction liquide-liquide.

## Patentansprüche

1. Verfahren zur Herstellung von Difluorethanol, **dadurch gekennzeichnet, dass** es eine Hydrierung eines Acetylhalogenids der folgenden Formel: 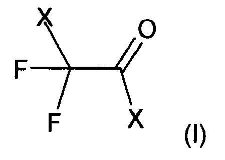 worin X für ein aus einem Chloratom und einem Bromatom ausgewähltes Halogenatom steht, in Gegenwart einer wirksamen Menge eines Katalysators, der mindestens ein Element der Gruppe VIII des Periodensystems umfasst, das auf einem festen anorganischen Träger mit sauren Eigenschaften, der aus Aluminiumoxid und natürlichen oder synthetischen Aluminosilikaten ausgewählt ist, abgeschieden ist, umfasst.

2. Verfahren nach Anspruch 1, **dadurch gekennzeichnet, dass** das eingesetzte Acetylhalogenid der Formel (I), worin X für ein Chloratom steht, entspricht.

3. Verfahren nach einem der Ansprüche 1 bis 2, **dadurch gekennzeichnet, dass** der Katalysator auf einem Metallelement basiert, das aus Ruthenium, Rhodium, Palladium, Osmium, Iridium und Platin ausgewählt ist.

4. Verfahren nach einem der Ansprüche 1 bis 3, **dadurch gekennzeichnet, dass** das Metallelement in Form eines feinverteilten Metalls oder in Form einer Verbindung, die in Gegenwart von Wasserstoff zum Metall reduziert wird, auf dem Träger abgeschieden sein kann.

5. Verfahren nach einem der Ansprüche 1 bis 4, **dadurch gekennzeichnet, dass** es sich bei dem Träger um α-, γ- oder η-Aluminiumoxid handelt.

6. Verfahren nach einem der Ansprüche 1 bis 4, **dadurch gekennzeichnet, dass** es sich bei dem Träger um einen sauren Ton handelt.

7. Verfahren nach einem der Ansprüche 1 bis 4, **dadurch gekennzeichnet, dass** es sich bei dem Träger um einen natürlichen oder synthetischen sauren Zeolith handelt.

8. Verfahren nach Anspruch 1, **dadurch gekennzeichnet, dass** es sich bei dem Katalysator um einen Katalysator auf Basis von Palladium und/oder Platin, das auf Aluminiumoxid abgeschieden ist, handelt.

9. Verfahren nach einem der Ansprüche 1 bis 8, **dadurch gekennzeichnet, dass** die Reaktion in der Gasphase durchgeführt wird.

10. Verfahren nach Anspruch 9, **dadurch gekennzeichnet, dass** die Reaktion bei einer Temperatur in einem Temperaturbereich von 150°C bis 350°C durchgeführt wird.

11. Verfahren nach einem der Ansprüche 9 bis 10, **dadurch gekennzeichnet, dass** die Reaktion unter einem Wasserstoffdruck zwischen 1 und 10 bar stattfindet.

12. Verfahren nach einem der Ansprüche 9 bis 11, **dadurch gekennzeichnet, dass** die Verweilzeit des Stoffstroms an der Katalysatorschüttung sehr kurz ist und zwischen weniger als 1 Sekunde bis 1 min variiert.

13. Verfahren nach einem der Ansprüche 9 bis 12, **dadurch gekennzeichnet, dass** die Reaktion gemäß der Festbett-Technik durchgeführt wird.

14. Verfahren nach Anspruch 13, **dadurch gekennzeichnet, dass** man die Katalysatorschüttung auf die Reaktionstemperatur bringt, den Wasserstoffstrom durchleitet und dann die Verbindung der Formel (I) einträgt.

15. Verfahren nach einem der Ansprüche 1 bis 7, **dadurch gekennzeichnet, dass** die Reaktion in der Flüssigphase durchgeführt wird.

16. Verfahren nach Anspruch 15, **dadurch gekennzeichnet, dass** der Wasserstoffdruck zwischen 1 und 50 bar liegt.

17. Verfahren nach einem der Ansprüche 15 bis 16, **dadurch gekennzeichnet, dass** die Reaktion bei einer Temperatur in einem Temperaturbereich von 20°C bis 150°C durchgeführt wird.

18. Verfahren nach einem der Ansprüche 15 bis 17, **dadurch gekennzeichnet, dass** die Reaktion in einem aus gegebenenfalls halogenierten aliphatischen, cycloaliphatischen und aromatischen Kohlenwasserstoffen ausgewählten organischen Lösungsmittel durchgeführt wird.

19. Verfahren nach einem der Ansprüche 15 bis 18, **dadurch gekennzeichnet, dass** die eingesetzte Hydrierkatalysatormenge, ausgedrückt in Katalysatorgewicht, bezogen auf das Gewicht der Verbindung der Formel (I), zwischen 0,5 und 20% variiert.

20. Verfahren nach einem der Ansprüche 1 bis 19, **dadurch gekennzeichnet, dass** man das Difluorethanol am Ende der Reaktion durch Destillation oder durch Flüssig-Flüssig-Extraktion gewinnt.

## Claims

1. Process for the preparation of difluoroethanol, **characterized in that** it comprises a hydrogenation, in the presence of an effective amount of a catalyst comprising at least one element from Group VIII of the Periodic Table deposited on a solid inorganic support having acid properties selected from alumina and natural or synthetic silicoaluminates, of an acetyl halide corresponding to the following formula: 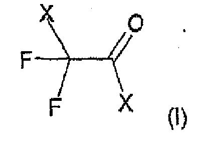 in said formula, X represents a halogen atom selected from a chlorine atom and a bromine atom.

2. Process according to Claim 1, **characterized in that** the acetyl halide employed corresponds to the formula (I) in which X represents a chlorine atom.

3. Process according to either of Claims 1 and 2, **characterized in that** the catalyst is based on a metal element selected from: ruthenium, rhodium, palladium, osmium, iridium, platinum.

4. Process according to one of Claims 1 to 3, **characterized in that** the metal element can be deposited on the support in the form of a finely divided metal or in the form of a compound which will be reduced to the metal in the presence of hydrogen.

5. Process according to one of Claims 1 to 4, **characterized in that** the support is α-, γ- or η-alumina.

6. Process according to one of Claims 1 to 4, **characterized in that** the support is an acid clay.

7. Process according to one of Claims 1 to 4, **characterized in that** the support is a acid natural or synthetic zeolite.

8. Process according to Claim 1, **characterized in that** the catalyst is a catalyst based on palladium and/or platinum deposited on alumina.

9. Process according to one of Claims 1 to 8, **characterized in that** the reaction is carried out in the vapor phase.

10. Process according to Claim 9, **characterized in that** the reaction is carried out at a temperature selected within a temperature range extending from 150°C to 350°C.

11. Process according to either of Claims 9 and 10, **characterized in that** the reaction takes place under a hydrogen pressure varying between 1 and 10 bar.

12. Process according to one of Claims 9 to 11, **characterized in that** the residence time of the material stream over the catalytic bed is very short and varies between less than one second to 1 min.

13. Process according to one of Claims 9 to 12, **characterized in that** the reaction is carried out according to the fixed bed technique.

14. Process according to Claim 13, **characterized in that** the catalytic bed is brought to the temperature of the reaction, the hydrogen stream is passed and then the compound of formula (I) is introduced.

15. Process according to one of Claims 1 to 7, **characterized in that** the reaction is carried out in the liquid phase.

16. Process according to Claim 15, **characterized in that** the hydrogen pressure is between 1 and 50 bar.

17. Process according to either of Claims 15 and 16, **characterized in that** the reaction is carried out at a temperature selected within a temperature range extending from 20°C to 150°C.

18. Process according to one of Claims 15 to 17, **characterized in that** the reaction is carried out in an organic solvent selected from halogenated or nonhalogenated aliphatic, cycloaliphatic or aromatic hydrocarbons.

19. Process according to one of Claims 15 to 18, **characterized in that** the amount of hydrogenation catalyst employed, expressed as weight of catalyst per weight of compound of formula (I), varies between 0.5 and 20%.

20. Process according to one of Claims 1 to 19, **characterized in that** the difluoroethanol is recovered at the end of the reaction by distillation or by liquid/liquid extraction.
